Europäisches Patentamt

European Patent Office

Office européen des brevets

(19)

(11) Publication number: 0 290 144
A1

(12) **EUROPEAN PATENT APPLICATION**

(21) Application number: 88303213.8

(22) Date of filing: 11.04.88

(51) Int. Cl.4: **C12Q 1/68 , C12N 15/00**

(30) Priority: 05.05.87 US 46127

(43) Date of publication of application:
09.11.88 Bulletin 88/45

(84) Designated Contracting States:
AT BE CH DE ES FR GB GR LI LU NL SE

(71) Applicant: **City of Hope**
**1500 East Duarte Road**
**Duarte California 91010(US)**

(72) Inventor: **Scanlon, Kevin Joseph**
**1520 Rodney Drive No. 209**
**Los Angeles California 90027(US)**

(74) Representative: **De Minvielle-Devaux, Ian**
**Benedict Peter et al**
**CARPMAELS & RANSFORD 43, Bloomsbury**
**Square**
**London WC1A 2RA(GB)**

(54) **Method of detecting incipient resistance to therapeutic agents in cancer patients.**

(57) A method for detecting incipient drug resistance to therapeutic agents in cancer patients, comprising determining an increase in the number of copies in the patient of at least one gene indicative of the incipient resistance.

FIG 1

# METHOD OF DETECTING INCIPIENT RESISTANCE TO THERAPEUTIC AGENTS IN CANCER PATIENTS

## BACKGROUND OF THE INVENTION

This invention relates to a method of detecting resistance to chemotherapeutic agents in cancer patients. Using this method, specific genes in a patient's cancer cells that are known to become amplified when cancer cells are resistant to chemotherapeutic agents are quantitatively measured. According to this invention, the determination of the increase in the number of specific gene copies or gene products in a patient indicates whether the patient has become resistant to the chemotherapeutic agents being used to treat the cancer.

Cis-diaminedichloroplatinum II (cisplatinum) is widely used in the treatment of carcinomas; yet, its mechanism of action remains unclear. Resistance to cisplatinum and other chemotherapeutic agents, such as 5-fluorouracil (5-FUra) is one of the main obstacles in cancer chemotherapy.

Recently, Scanlon, K.J., et al., Proc.Natl.Acad.Sci. USA 83:8923 (1986) have pointed to the thymidylate synthase (dTMP synthase) cycle, which is the pathway for synthesis of thymidine precursors, as an additional site of action of cisplatinum. Co-pending application Serial No. 859,065 filed May 2, 1986, incorporated herein by reference, describes the inhibition of dTMP synthase in human cancer cells by sequential administration, first, of cisplatinum and then of 5-FUra by causing a metabolite of 5-FUra to covalently bind to dTMP synthase and 5-fluorodeoxyuridylic acid FdUMP. This combination of drugs used in vivo has been shown to achieve very favorable responses against human neoplasms such that sequential administration of cisplatinum and then 5-FUra has a synergistic effect.

As an additional consideration, cellular oncogenes have been implicated in the control of normal cell growth and proliferation. The H-ras oncogene product is a g protein and may play a role in signal transduction. The myc and fos genes encode nuclear proteins induced following serum addition to quiescent cells. It possible that the action of these proteins leads to DNA synthesis.

It has been determined that human carcinoma cells become resistant to cisplatinum in vitro and in vivo due to the activity elevation of dTMP synthase and dihydrofolate reductase (DHFR) and a concomitant increase in the mRNA and gene expression of dTMP synthase and DHFR. Further, resistance to chemotherapeutic agents in humans has been found to cause an increase in the number of gene copies of the oncogenes c-fos, c-H-ras, and c-myc.

In a clinical setting, a rapid and practical detection of the increase in the number of copies of the genes of dTMP synthase, DHFR, c-fos, c-H-ras, and c-myc would lead to rapid diagnosis of a patient's early resistance to chemotherapeutic agents. This invention provides a rapid method of detecting a patient's early resistance to chemotherapeutic agents by measuring the increased expression of these genes.

Saiki, R.K., et al., Science 230:1350 (1985) shows that small amounts of DNA samples, undetectable with standard nucleic acid hybridization methods, can be specifically detected after amplification achieved by repeated synthesis of nucleic acid sequences bound by oppositely oriented primers.

According to the method of the present invention, this technique known as the polymerase chain reaction is modified to provide for the amplification of an RNA template. Specifically, by beginning with m-RNA that codes for the DNA of dTMP synthase, DHFR, c-fos, c-H-ras, and c-myc, these specific genes are exponentially amplified in vitro, and compared to a control. Identification of the specific genes to be amplified is essential to this assay.

## SUMMARY OF THE INVENTION

In general, the invention comprises a method for detecting incipient drug resistance to therapeutic agents in cancer patients, comprising determining an increase in the number of copies in the patient of at least one gene indicative of the incipient resistance. The increase in the number of gene copies is determined by comparing an amplification of the messenger RNA sequence that codes for at least one indicative gene with a control. The genes indicative of incipient resistance are selected from the group consisting of dTMP synthase, dihydrofolate reductase, c-fos, c-H-ras, and c-myc.

Other advantages and features of the invention will be apparent from the following description of the preferred embodiments thereof, and from the claims.

## DESCRIPTION OF THE DRAWINGS

Fig. 1 is a flow chart depicting the steps of the assay of the invention.

Fig. 2a depicts the oligoprimers for dTMP synthase.

Fig. 2b depicts the oligoprimers for DHFR.

Fig. 3a shows Southern analysis of labeled cDNA for dTMP synthase hybridized to amplification products formed by using four different oligomer primers for dTMP synthase.

Fig. 3b shows Southern analysis of amplified mRNA for dTMP synthase from sensitive (A2780S) and resistant (A2780DDP) cells.

## DESCRIPTION OF THE PREFERRED EMBODIMENT

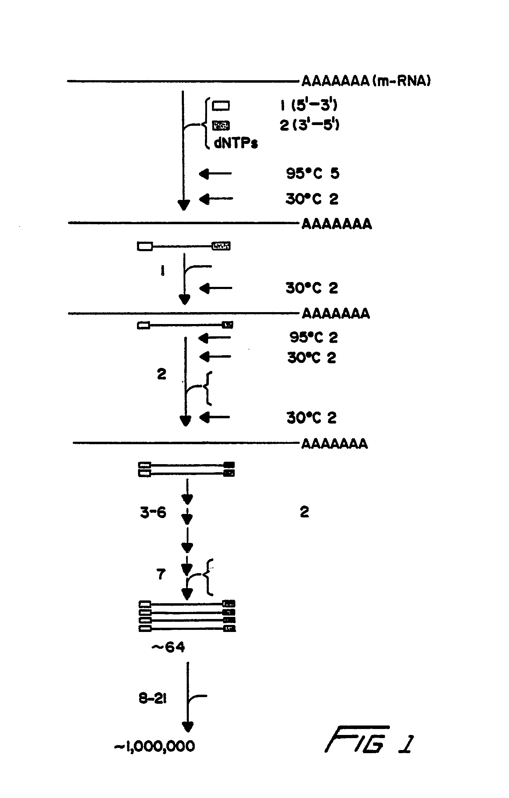

The illustrated procedure yields exponential in vitro amplification of 181 base nucleic acid sequences of a specific gene. A schematic diagram outlining the steps of the assay of the invention is shown in Fig. 1.

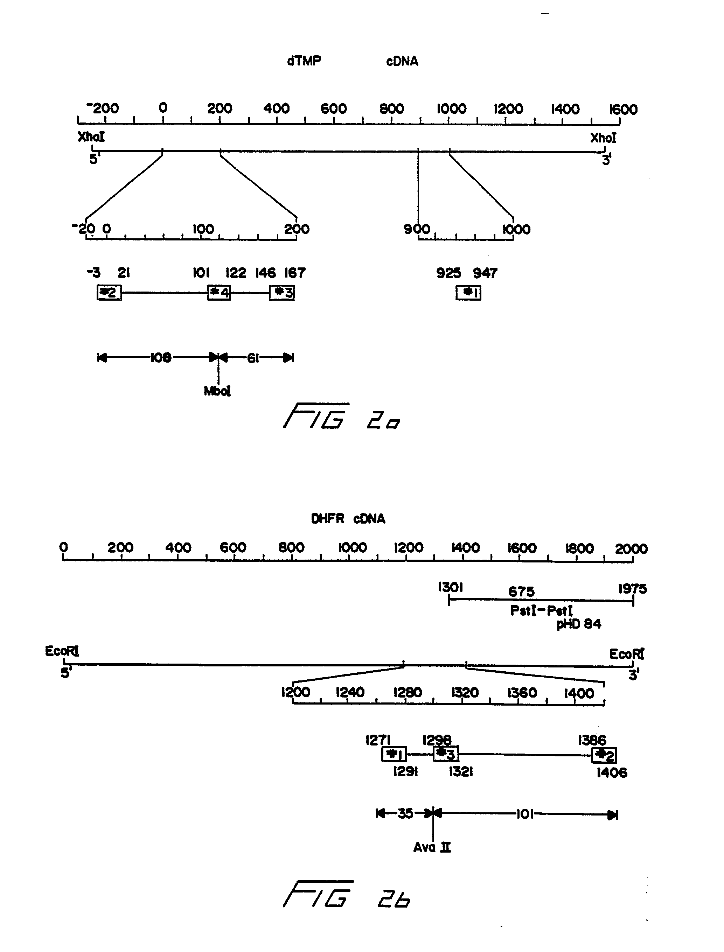

This assay requires two converging, preferably about twenty base, oligoprimers oriented in opposite directions, for the 5' and 3' ends of the gene sequence to be analyzed. These oligoprimers may be synthesized by conventional methods. The oligoprimers for dTMP synthase are shown in Fig. 2a; the oligoprimers for DHFR are shown in Fig. 2b.

More specifically for dTMP synthase, TS-2 is the 3'-5' oligoprimer complementary to DNA (bases -3-21) having the sequence GCC ATG CCT GTG GCC GGC TCG GAG, and TS-3 is the 5'-3' primer complementary to mRNA (bases 146-167) having the sequence AGG GTG CCG GTG CCC GTG CGG T. The probe for identifying the TS sequence (base 101-122) is denoted TS-4 and has the sequence AGG ATG TGT TG G A T C TGC CCC A.

*MboI restriction enzyme site

DHFR-1 IS THE 3'-5' oligoprimer complementary to DNA (bases 1271-1291) having the sequence GAC CGC GCG TTC TGC TGT AAC. DHFR-2 is the 5'-3' oligoprimer complementary to mRNA (bases 1386-1406) having the sequence GAG CGG TGG CCA GGG CAG GTC. The probe for identifying the sequence, DHFR-3, has the sequence ACA GCA GCG GGA GGA C C T CCG AGC.

*Ava II restriction enzyme site

In the following examples the nucleic acid sequence of human dTMP synthase was amplified according to the assay of the invention.

## Example 1

Cycle 1 of the assay of this invention was started by combining messenger RNA (mRNA) from drug resistant tumor cells of a patient with 1X amplification buffer (10 mM tris-HCl, pH 7.5; 10 mM MgCl$_2$; 66 mM NaCl; 1 mM dithiothereitol), a large molar excess (1.0 μM) of both the 5' and 3' primers of complementary mRNA (TS-3 and TS-2), and a large molar excess (1.5 mM) of the four deoxynucleoside triphosphates such that the final reaction volume was 100 μl. The mRNA was denatured by heating for 5 minutes at 95°C, centrifuged for 5 seconds, and cooled to 30°C. Cycle 1 of the reaction was completed by adding 1.0 μl of reverse transcriptase (2.0 units, Bio Rad Research Laboratories, Inc.) diluted in amplification buffer, and incubating the solution for 2 minutes at 30°C. At this point of the reaction, and during each subsequent reaction cycle, each oligonucleotide primer on the original mRNA template was extended to produce one new single strand dTMP synthase DNA molecule of 181 bases.

After completion of the cycle 1 reaction, the solution was denatured by heating at 95°C for 2 minutes, centrifuged, and cooled to 30°C as in cycle 1. Next, 0.5 units of Klenow DNA polymerase I (Klenow) (Boehringer Mannheim) and a further amount of reverse transcriptase were added, and the solution was heated at 30°C for 2 minutes to end cycle 2. At this point of the reaction, two copies of DNA were obtained.

As seen in Fig. 1, cycles 3-6 were performed by repeating the steps of cycle 2. In cycle 7, 0.45 ug of ribonuclease A (RNase) was added along with Klenow to eliminate the RNA complexity and to enhance primer hybridization. All cycles from the eighth onward were carried out by the addition of Klenow only. At the end of 21 cycles, there were approximately 1,000,000 copies of dTMP synthase DNA.

As stated above, performing each cycle of the assay of this invention produced one new single strand DNA molecule, which in this example was the 181 base dTMP synthase DNA. The single strand DNA molecules that were produced by the assay of this invention acted as templates for one or the other of the oligonucleotide primers during sebsequent cycles of the assay. Extension of thes oligonucleotides by Klenow produced molecules of dTMP synthase that were 181 bases long. As a result, a chain reaction was sustained which resulted in the accumulation of, in this instance, the specific 181-base pair double stranded DNA at an exponential rate as compared to the number of cycles of the assay performed.

## Example 2

The assay reaction described in Example 1 was carried out four different times, each reaction starting with 1 μg of dTMP synthase mRNA and one pair of oligoprimers comprised of the four different oligoprimers for the 169 base pair dTMP synthase fragment as shown in Fig. 2. The four pairs of oligoprimers used were: TS-1 and TS-3; TS-2 and TS-3; TS-1 and TS-4; and TS-2 and TS-4.

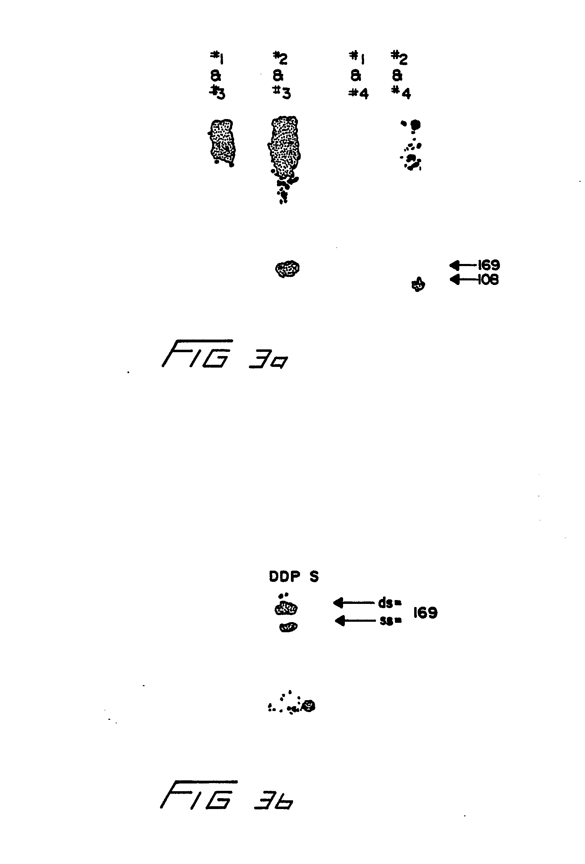

To test the four different amplification products obtained, TS-4 probe labeled with $^{32}$P was hybridized to the products and Southern blot analyses were performed as described by Southern, E.M., J. Mol. Biol. 98:503 (1975). The results of the Southern blot analysis are shown in Fig. 3a.

Only oligoprimers TS-2 and TS-3 optimally amplified the dTMP synthase gene fragment. Oligoprimers TS-2 and TS-4 amplified the sequence to a lesser extent, while the combinations of primers TS-1 and TS-3, and TS-1 and TS-4 were not successful. The selection of oligoprimers for a given gene sequence that is to be amplified by the assay of the invention is critical.

There was an exponential growth of the 181-base pair fragment which began with 1 μg of human dTMP synthase mRNA and TS-2 and TS-3 oligoprimers. After 10 cycles, the target sequence of dTMP synthase was greatly amplified. Amplifying 1 μg total human dTMP synthase mRNA produced approximately $5 \times 10^{-19}$ moles of the target sequence from one copy of gene and produced a 1,000,000-fold increase of this fragment after 20 cycles of the assay reaction.

## Example 3

The dTMP synthase mRNA from human ovarian carcinoma cells A2780S (sensitive to chemotherapeutic agents) and A2780DDP (resistant to chemotherapeutic agents) grown in vitro were amplified using oligomer primers TS-2 and TS-3. The reaction was started with 1 μg of mRNA for each cell line. However, a far lesser quantity of mRNA, e.g., 100-fold less, could also have been used. The results of Southern bolt analysis of the amplification products as described in Example 2 are shown in Fig. 3b, further illustrating the use of the assay as a method of determining resistance to chemotherapeutic agents in cancer patients. In this example, the amplified gene product from the DDP (resistant) cells shows up as both double strand (ds) and single strand (ss) product. By comparison; no detectable 181 base product was produced at all for the S (sensitive) cells.

These results are consistent with previous findings that resistance to chemotherapeutic agents results in an increase in the amount of mRNA and gene expression of dTMP synthase, DHFR, and the H-ras, c-fos, and c-myc oncogenes in carcinoma cells. Therefore amplifying mRNA that codes for these enzymes or oncogenes from even one carcinoma cell of a patient, and comparing the amount of amplification product with a control such as DNA from white blood cells, a rapid and accurate diagnosis of a cancer patient's chemotherapy progress can be made.

For example, a patient's cell that normally contained one copy of m-RNA for one of the enumerated enzymes or oncogenes, but that later contained five copies of the same m-RNA when the patient had become resistant to chemotherapeutic agents, the amount of "drug resistant" genes that would be amplified via the assay of this invention would five times greater than the amount of amplified genes in the normal (control) cells. Further, if the results of Fig. 3b showing a large increase in the expression of the gene for dTMP synthase were from a cancer patient, it would be immediately apparent that the patient had become resistant to the chemotherapeutic agents being administered. The patient could then be switched to different chemotherapeutic agents. The progress of the patient under the new regimen could be similarly monitored by daily analysis of the patient's carcinoma cells according to the methods of the present invention.

Other therapeutic agents besides cisplatinum and 5-FUra to which the methods of this invention may be used to detect resistance thereto are analogs of cisplatinum, fluoropyrimidines other than 5-FUra and analogs thereof, and antifolates such as methotrexate and analogs thereof.

The carcinoma cells to be analyzed by the methods herein described can be obtained by biopsy in the case of solid tumors, or in the case of cancer of the blood, blood samples can be obtained.

The methods of this invention can be used on a daily or less frequent basis to detect a patient's early resistance to chemotherapeutic agents.

## Claims

1. A method for detecting incipient drug resistance to therapeutic agents in cancer patients, comprising:

determining an increase in the number of copies in a sample from said patient of at least one gene indicative of said incipient resistance.

2. The method of claim 1 wherein the increase in the number of gene copies is determined by comparing an amplification of the messenger RNA sequence that codes for at least one indicative gene with a control.

3. The method of claim 1 in which the gene is selected from the group consisting of dTMP synthase, dihydrofolate reductase, c-fos, c-H-ras, and c-myc.

4. The method of claim 1 in which the gene is dTMP synthase.

5. The method of claim 1 in which the gene is dihydrofolate reductase.

6. The method of claim 1 in which the gene is c-fos.

7. The method of claim 1 in which the gene is c-H-ras.

8. The method of claim 1 in which the gene is c-myc.

9. A method for detecting incipient resistance in cancer patients to a therapeutic agent selected from the group consisting of cis-diaminedichloroplatinum II, analogs of cisplatinum, 5-fluorouracil, methotrexate, fluoropyrimidines and analogs thereof; and antifolates and analogs thereof comprising:

determining an increase in the number of copies in a sample from said patient of at least one gene indicative of said incipient resistance.

10. The method of claim 9 wherein the increase in the number of gene copies is determined by comparing an amplification of the messenger RNA sequence that codes for at least one indicative gene with a control.

11. The method of claim 9 in which the gene is selected from the group consisting of dTMP synthase, dihydrofolate reductase, c-fos, c-H-ras, and c-myc.

0 290 144

—————————————— AAAAAAA (m-RNA)

$1 (5'—3')$

$2 (3'—5')$

dNTPs

95°C 5

30°C 2

————————————————— AAAAAAA

1

30°C 2

————————————————— AAAAAAA

95°C 2

30°C 2

2

30°C 2

————————————————— AAAAAAA

3-6          2

7

~64

8-21

~1,000,000

FIG 1

FIG 2a

FIG 2b

FIG 3a

FIG 3b

European Patent Office

# EUROPEAN SEARCH REPORT

Application Number

EP 88 30 3213

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-8 200 158 (SALK INSTITUT FOR BIOLOGICAL STUDIES) * Page 3, lines 13-34; page 9, line 1 - page 11, line 12 * | 1 | C 12 Q 1/68 C 12 N 15/00 |
| X | WO-A-8 303 259 (TRUSTEES OF COLUMBIA UNIVERSITY) * Page 2, line 26 - page 3, line 4; page 5, lines 3-20; page 9, line 31 - page 10, line 2; page 42, line 3 - page 43, line 25 * | 1,3,5, 11 | |
| X | US-A-4 656 134 (G.M. RINGOLD) * Column 1, lines 49-59; column 8, lines 25-47; column 10, lines 16-66 * | 1,3,5, 11 | |
| X | US-A-4 442 203 (A.J. VARSHAVSKY) * Column 2, line 62 - column 3, line 36; column 6, examples 1,2; column 8, line 59 - column 12, line 5 * | 1,3,5,9 ,11 | |
| X | EP-A-0 201 184 (CETUS CORP.) * Column 3, lines 48-55; column 20, lines 5-15; columnn 44, lines 20-50; column 45, line 15 - column 46, line 8 * | 2,10 | TECHNICAL FIELDS SEARCHED (Int. Cl.4) C 12 Q C 12 N 15 |
| X | SCIENTIFIC AMERICAN, vol. 243, no. 5, November 1980, pages 50-59, New York, US; R.T. SCHIMKE: "Gene amplification and drug resistance" * Page 50, column 2, line 24 - page 52, column 3, line 17 * -/- | 1,3,5,9 ,11 | |

The present search report has been drawn up for all claims

| Place of search | Date of completion of the search | Examiner |
|---|---|---|
| THE HAGUE | 12-08-1988 | VAN BOHEMEN C.G. |

EPO FORM 1503 03.82 (P0401)

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 108 564 (REGENTS OF THE UNIVERSITY OF CALIFORNIA) <br> * Page 5, table 1; page 6, lines 21-26; page 10, lines 1-23; page 11, lines 4-32; page 33, lines 3-32 * | 1-11 | |
| Y | SCIENCE, vol. 202, 8th December 1978, pages 1051-1055, AAAS, Washington D.C., US; R.T. SCHIMKE et al.; "Gene amplification and drug resistance in cultured murine cells" <br> * Page 1051, summary; page 1051, column 1, line 22 - page 1053, column 3, line 13 * | 1-11 | |
| | | | **TECHNICAL FIELDS SEARCHED (Int. Cl.4)** |

The present search report has been drawn up for all claims

| Place of search | Date of completion of the search | Examiner |
|---|---|---|
| THE HAGUE | 12-08-1988 | VAN BOHEMEN C.G. |